# EUROPEAN PATENT SPECIFICATION

(11) **EP 2 000 453 B1**
(45) Date of publication and mention of the grant of the patent: **03.12.2014**
(21) Application number: 07715231.2
(22) Date of filing: 06.03.2007
(51) Int. Cl.: C07C 29/16, C07C 31/125, C07C 31/135, C07C 33/12, C07B 41/02

(54) **METHOD FOR PRODUCING ALCOHOL BY USING CARBON DIOXIDE AS RAW MATERIAL**
VERFAHREN ZUR HERSTELLUNG VON ALKOHOL DURCH VERWENDUNG VON KOHLENDIOXID ALS ROHSTOFF
PROCEDE DE PRODUCTION D'UN ALCOOL UTILISANT DU DIOXYDE DE CARBONE EN TANT QUE MATIERE PREMIERE

(30) Priority: 28.03.2006 JP 2006087788
(43) Date of publication of application: 10.12.2008
(73) Proprietor: Hitachi Chemical Company, Ltd., Tokyo 100-6606 (JP); National Institute of Advanced Industrial Science and Technology, Tokyo 100-8921 (JP)
(72) Inventor: TOMINAGA, Kenichi, Tsukuba-shi, Ibaraki 3058565 (JP); SATO, Kazuhiko, Tsukuba-shi, Ibaraki 3058565 (JP); KAMEI, Junichi, Tokyo 100-6606 (JP); MARUYAMA, Tetsushi, Tokyo 100-6606 (JP); KOBAYASHI, Akihiro, Tokyo 100-6606 (JP)
(74) Representative: Hoffmann Eitle
(86) International application number: PCT/JP2007/054263
(87) International publication number: WO 2007/111091

(56) References cited:
- JP-A- 55 118 429
- JP-A- 2001 233 795
- JP-A- 2004 091 331
- JAASKELAINEN S. ET AL.: 'The use of carbon dioxide in ruthenium carbonyl catalyzed 1-hexene hydroformylation promoted by alkali metal and alkaline earth salts' APPLIED CATALYSIS A: GENERAL vol. 247, 2003, pages 95 - 100, XP004434429
- TOMINAGA K.: 'An environmentally friendly hydroformylation using carbon dioxide as a reactant catalyzed by immobilized Ru-complex in ionic liquids' CATALYSIS TODAY, [Online] vol. 115, 24 March 2006, pages 70 - 72, XP005451294 Retrieved from the Internet: <URL:http://www.sciencedirect.com>

## Description

### TECHNICAL FIELD

The invention relates to the method for producing an alcohol. In particular, the invention relates to the method for producing an alcohol by hydroformylation using carbon dioxide as a raw material.

### BACKGROUND

Today, as a common method for producing an alcohol, hydration or hydroformylation of a raw material organic compound having an unsaturated carbon bond is known. In the former method, however, a secondary or tertiary alcohol tends to be produced due to the Markovnikov's rule. Therefore, in order to produce a primary alcohol, it is required to use a hydroformylation reaction. For this reason, the hydroformylation reaction has become one of the most important processes in the chemical industry, and is used for producing more than six millions of chemical products a year in the world.

However, the hydroformylation reaction uses as a raw material a large amount of carbon monoxide which is extremely toxic. In order to conduct the reaction on an industrial scale, heavy investments cannot be avoided to ensure safety management and environment protection.

In order to solve this problem, the inventors have developed a novel method of hydroformylation in which a ruthenium compound is used as a catalyst to enable carbon dioxide, which is much safer not only to the human body but also to the environment, to be used as a raw material (Patent Document 1). Furthermore, the inventors have developed a method in which a common raw material compound is selectively hydroformylated with carbon dioxide to produce an alcohol by using a catalyst obtained by dispersing a ruthenium compound in a non-aqueous ionic liquid composed of an organic or inorganic salt which becomes a liquid around room temperature (Patent Document 2). As apparent from the above, production of an alcohol by hydroformylation using carbon dioxide directly as a raw material is unique both at home and abroad.

Also known from Document 3 is a process for preparing alcohols from alkenes with carbon dioxide and hydrogen in the presence of a clustered ruthenium catalyst and a Bronsted acid, HCl (see Table 1). The reaction is carried out at a temperature of 150 °C and a pressure of 60 bar.
Patent Document 1: JP-A-2001-233795
Patent Document 2: JP-A-2004-091331
Document 3:JAASKELAINEN S. ET AL.: "The use of carbon dioxide in ruthenium carbonyl catalyzed 1-hexene hydroformylation promoted by alkali metal and alkaline earth salts", APPLIED CATALYSIS A: GENERAL

The object of the invention is to provide a method for producing an alcohol quickly and efficiently by using an organic compound having an unsaturated carbon bond, carbon dioxide and hydrogen as raw materials.

### DISCLOSURE OF THE INVENTION

As a result of intensive studies, the inventors have found that an intended alcohol can be produced quickly by using a catalyst system comprising a ruthenium compound and a Brønsted acid containing phosphor. According to this method, only the speed of intended hydroformylation can be increased without increasing the speed of hydrogenation of a raw material compound, which is a side reaction, whereby the yield and selectivity of the intended product are also improved.

According to the invention, the following method for producing an alcohol can be provided.
1. A method for producing an alcohol which comprises hydroformylation of an organic compound having an unsaturated carbon bond with carbon dioxide and hydrogen by using a catalyst system comprising a ruthenium compound and a Brønsted acid containing phosphor.
2. The method for producing an alcohol according to 1, wherein the ruthenium compound is a clustered ruthenium complex.
3. The method for producing an alcohol according to 1 or 2, wherein the catalyst system further comprises a halide salt.
4. The method for producing an alcohol according to 3, wherein the halide salt is a non-aqueous ionic liquid.
5. The method of any of claims 1-4, wherein the hydroformylation reaction is performed at a temperature of 100-180°C and at a pressure of 1-50 MPa.
6. The method of any of claims 1-5, wherein the organic compound is a compound having two or more unsaturated carbon bonds, and a polyvalent alcohol is produced by using the organic compound.

The invention can provide the method for producing an alcohol quickly and efficiently by using an organic compound having an unsaturated carbon bond, carbon dioxide and hydrogen as raw materials.

### BEST MODE FOR CARRYING OUT THE INVENTION

In the method of producing an alcohol according to the invention, an organic compound having an unsaturated carbon bond is hydroformylated with carbon dioxide and hydrogen, and a catalyst system comprising a ruthenium compound and a Brønsted acid containing phosphor is used during the hydroformylation.

There are no particular restrictions on the organic compound having an unsaturated carbon bond to be used in the invention insofar as it is a compound having an unsaturated carbon bond, and examples include an aliphatic chain unsaturated compound, an aliphatic cyclic unsaturated compound and an aromatic compound. Here, the unsaturated carbon bond may be present either on the terminal or the inside of a molecule. Furthermore, an organic compound having a plurality of unsaturated carbon bonds may also be used. It is also possible to produce a polyvalent alcohol having a plurality of hydroxymethyl groups (-CH₂OH) by using the organic compound having a plurality of unsaturated carbon bonds as a raw material. In these unsaturated compounds, the hydrogen atom within the molecule may be substituted with one or more groups selected from an alkyl group, cyclic aliphatic group, aromatic group, heterocyclic group, carbonyl group, alkoxy group, cyano group, amino group, amide group, nitro group, halogen, and phosphor-containing substituent.

Examples of the aliphatic chain unsaturated compound include ethylene, propylene, butylene, pentene, hexene, heptene, octene, nonene, decene, undecene, dodecene, tridecene, tetradecene, pentadecene, hexadecene, heptadecene, octadecene, nonadecene, butadiene, pentadiene, hexadiene, heptadiene, octadiene, nonadiene, hexanetriene, heptatriene, octatriene, and the isomers and derivatives thereof. Examples of the aliphatic cyclic unsaturated compounds include cyclopentene, cyclohexene, cycloheptene, cyclooctene, cyclopentadiene, cyclohexadiene, cycloheptadiene, cyclooctadiene, tetrahydroindene, methyltetrahydroindene, norbornene, norbornadiene, methylvinylnorbornene, dicyclopentadiene, methyldicyclopentadiene, tricyclopentadiene, tetracyclopentadiene, and the isomers and derivatives thereof. Examples of the aromatic chain unsaturated compound include stylene, stilbene, triphenylethylene, tetraphenylethylene, and the derivatives thereof. Examples of the aromatic cyclic unsaturated compound include indene, dihydronaphthalene, indole and the derivatives thereof.

The raw material gas used in the invention is a mixed gas containing hydrogen and carbon dioxide as main components. The content of the carbon dioxide is preferably 10 to 95 vol%, more preferably 50 to 80 vol%. The content of the hydrogen is preferably 5 to 90 vol%, more preferably 20 to 50 vol%. These gases may be supplied in the form of a mixed gas or may be supplied separately. If the total content of hydrogen exceeds 90%, significant hydrogenation of the raw material occurs. When the hydrogen content is 5% or less, the reaction speed may be significantly lowered. While carbon monoxide may not necessarily be mixed in the raw material gas, mixing of carbon monoxide poses no problems.

There are no restrictions on the ruthenium compound insofar as it contains ruthenium. Preferable examples of the ruthenium compound include clustered ruthenium compounds such as Ru₂(CO)₆Cl₄, Ru₃ (CO)₁₂, H₄Ru₄(CO)₁₂, H₂Ru₆(CO)₁₈, and H₂Ru₆C(CO)₁₆. The clustered compound may be obtained by clusterizing a raw material, a mononuclear ruthenium compound such as RuCl₃, RuCl₂(C₈H₁₂), Ru (CO)₃(C₈H₈), Ru (CO)₃(C₈H₁₂), Ru(C₈H₁₀) (C₈H₁₂) before or during the reaction. Common methods for clusterization include heating after the addition of carbon monoxide and heating after the addition of formic acid.

The amount of the ruthenium compound is preferably 1/10000 to 1 equivalent, more preferably 1/1000 to 1/50 equivalent, relative to the amount of the raw material compound. If the amount of the ruthenium compound is less than 1/10000 equivalent, the reaction tends to proceed extremely slowly. If the amount of the ruthenium compound exceeds 1 equivalent, the raw material compound may be hydrogenated before the reaction of the carbon dioxide.

The above-mentioned acid is a phosphor-containing Bronsted acid. Of these, a phosphor-containing acid such as phosphoric acid, alkylphosphoric acid and phenylphosphoric acid is preferable.

The amount of the acid added is 0.1 to 100 equivalents, preferably 1 to 10 equivalents relative to the amount of the ruthenium compound, for example. If the amount of the acid is less than 0.1 equivalent relative to the amount of the ruthenium compound, there are almost no effects of promoting the reaction by the addition of an acid. If the amount exceeds 100 equivalents, productivity may be lowered significantly.

Preferably, a halide salt is added to the catalyst system of the invention. As for the cation used in the halide salt, either an inorganic ion or an organic ion may be used.

Preferred examples of the halide salt include chloride salts, bromide salts and iodide salts.

The halide salt is added in an amount of 1 to 1000 equivalents relative to the ruthenium compound, for example.

Examples of the inorganic ion used in the halide salt include lithium, sodium, potassium, rubidium, cesium, calcium and strontium. Examples of the organic ion include tetramethylammonium, tetraethylammonium, tetrapropylammonium, tetrabutylammonium, tetrapentylammonium, tetrahexylammonium, tetraheptylammonium, tetraoctylammonium, benzyltrimethylammonium, benzyltriethylammonium, benzyltributylammonium, tetramethylphosphonium, tetraethylphosphonium, tetraphenylphosphonium, benzyltriphenylphosphonium, and bis(triphenylphosphine)iminium.

The halide salt may not necessarily be a solid salt. Preferably, the halide salt is a non-aqueous ionic liquid containing a halide ion which becomes a liquid around room temperature or in a temperature range of 100°C or less.

Examples of the cation to be used as the non-aqueous ionic liquid include 1-ethyl-3-methylimidazolium, 1-propyl-3-methylimidazolium, 1-butyl-3-methylimidazolium, 1-pentyl-3-methylimidazolium, 1-hexyl-3-methylimidazolium, 1-hepthyl-3-methylimidazolium, 1-octyl-3-methylimidazolium, 1-decyl-3-methylimidazolium, 1-dodecyl-3-methylimidazolium, 1-tetradecyl-3-methylimidazolium, 1-hexadecyl-3-methylimidazolium, 1-octadecyl-3-methylimidazolium, 1-ethyl-2,3-dimethylimidazolium, 1-butyl-2,3-dimethylimidazolium, 1-hexyl-2,3-dimethylimidazolium, 1-ethylpyridinium, 1-butylpyridinium, 1-hexylpyridinium, 8-methyl-1,8-diazabicyclo[5.4.0]-7-undecene, 8-ethyl-1,8-diazabicyclo[5.4.0]-7-undecene, 8-propyl-1,8-diazabicyclo[5.4.0]-7-undecene, 8-butyl-1,8-diazabicyclo[5.4.0]-7-undecene, 8-pentyl-1,8-diazabicyclo[5.4.0]-7-undecene, 8-hexyl-1,8-diazabicyclo[5.4.0]-7-undecene, 8-heptyl-1,8-diazabicyclo[5.4.0]-7-undecene, and 8-octyl-1,8-diazabicyclo[5.4.0]-7-undecene. These halide salts may be used either alone or in combination of two or more.

It is preferred that the hydroformylation reaction be conducted in a temperature range of preferably about 100°C to 180°C, more preferably 120°C to 160°C. In a temperature range lower than 100°C, carbon dioxide may not be reacted. In a temperature range higher than 180°C, only hydrogenation of an unsaturated bond may tend to occur.

The hydroformylation reaction is conducted preferably under a pressure of 1 to 50MPa, more preferably 2 to 15MPa. If the pressure is less than 1MPa, the reaction may be slow. If the pressure exceeds 50MPa, further effects of promoting the reaction cannot be attained.

In the production method of the invention, a solvent may be used in a reaction system, for example. There are no restrictions on the usable solvent insofar as it can dissolve reaction raw materials. Preferable examples of the solvent include n-pentane, n-hexane, n-heptane, cyclohexane, benzene, toluene, o-xylene, p-xylene, m-xylene, ethylbenzene, cumene, tetrahydrofuran, N-methylpyrrolidone, dimethylformamide, dimethylacetoamide, dimethylimidazolidinone, ethylene glycol dimethyl ether, diethylene glycol dimethyl ether and triethylene glycol dimethyl ether. If a solvent is used, it is preferred that the solvent be used in such an amount it allows the concentration of the raw material compound to be 0.1 vol% or more, preferably 1.0 vol% or more.

### EXAMPLES

### Example 1

### [Hydroformylation of cyclohexene when phosphoric acid is added]

In a stainless steel- made pressurized reaction apparatus having an inner volume of 50 ml, 0.1 mmol of Ru₃(CO)₁₂ as the ruthenium compound, 0.5 mmol of bis(triphenylphosphine)iminium chloride as the halide salt, 0. 5 mmol of phosphoric acid as the acid, 20. 0 mmol of cyclohexene as the raw material organic compound and 5.0 mL of tetrahydrofuran as the solvent were put at room temperature, and stirred to dissolve. Thereafter, carbon dioxide and hydrogen were introduced with stirring under pressure of 4MPa and 4MPa, respectively, and retained at 140°C for 10 hours. Subsequently, the reaction apparatus was cooled to room temperature, and the pressure was released to extract the remaining organic phase. The extracted organic phase was analyzed by gas chromatography. The conversion ratio of cyclohexene was 86%. Cyclohexane methanol was formed as an alcohol in a yield of 68%, and cyclohexane was formed as a hydrogenated product in a yield of 11%.

### Example 2

### [Hydroformylation of cyclohexene when phenylphosphoric acid is added]

A reaction was conducted in the same manner as in Example 1, except that phenylphosphoric acid was used instead of phosphoric acid. The conversion ratio of cyclohexene was 94%. Cyclohexane methanol was formed in a yield of 77% and cyclohexane was formed as a hydrogenated product in a yield of 9%.

### Example 3

### [Hydroformylation of cyclohexene when phenylphosphonic acid is added]

A reaction was conducted in the same manner as in Example 1, except that phenylphosphonic acid was used instead of phosphoric acid. The conversion ratio of cyclohexene was 96%. Cyclohexane methanol was formed in a yield of 66% and cyclohexane was formed as a hydrogenated product in a yield of 7%.

### Example 4 (Comparative)

### [Hydroformylation of cyclohexene when acetic acid is added]

A reaction was conducted in the same manner as in Example 1, except that acetic acid was used instead of phosphoric acid. The conversion ratio of cyclohexene was 88%. Cyclohexane methanol was formed in a yield of 40% and cyclohexane was formed as a hydrogenated product in a yield of 28%.

### Example 5 (Comparative)

### [Hydroformylation of cyclohexene when trifluoroacetic acid is added]

A reaction was conducted in the same manner as in Example 1, except that trifluoroacetic acid was used instead of phosphoric acid. The conversion ratio of cyclohexene was 94%. Cyclohexane methanol was formed in a yield of 44% and cyclohexane was formed as a hydrogenated product in a yield of 39%.

### Example 6 (Comparative)

### [Hydroformylation of cyclohexene when phenylboric acid is added]

A reaction was conducted in the same manner as in Example 1, except that phenylboric acid was used instead of phosphoric acid. The conversion ratio of cyclohexene was 84%. Cyclohexane methanol was formed in a yield of 40% and cyclohexane was formed as a hydrogenated product in a yield of 33%.

### Comparative Example 1

### [Hydroformylation of cyclohexene without addition of an acid]

A reaction was conducted in the same manner as in Example 1, except that phosphoric acid was not added. The conversion ratio of cyclohexene was 77%. Cyclohexane methanol was formed in a yield of 38% and cyclohexane was formed as a hydrogenated product in a yield of 32%.

The above results show that, by the addition of an acid to a conventional ruthenium compound catalyst, the raw material conversion ratio and the yield of an alcohol, i.e., the speed of alcohol formation reaction, can be improved. The above results also show that, of the acids, a phosphor-containing acid is particularly effective.

### Example 7

### [Hydroformylation of 1-hexene when phenylphosphoric acid is added]

A reaction was conducted in the same manner as in Example 2, except that 1-hexene was used as the raw material organic compound. The conversion ratio of 1-hexene was 91%. Heptanol was formed in a yield of 66% and hexane was formed as a hydrogenated product in a yield of 13%.

### Comparative Example 2

### [Hydroformylation of 1-hexene without addition of an acid]

A reaction was conducted in the same manner as in Example 7, except that phenylphosphoric acid was not added. The conversion ratio was 70%. Heptanol was formed in a yield of 28%, heptanal was formed in a yield of 19% and hexene was formed as a hydrogenated product in a yield of 17%.

The above results show that, irrespective of the structure of the raw material organic compound (cyclic or chain), the speed of the hydroformylation reaction is increased by the addition of an acid, and the yield of an alcohol can be improved.

### Example 8

### [Hydroformylation of cyclohexene when a non-aqueous ionic liquid is used as the halide salt and phenylphosphoric acid is added]

A reaction was conducted in the same manner as in Example 2, except that 5.0 mmol of 1-butyl-3-methylimidazolium chloride was used as the halide salt and 5.0 mL of toluene was used as the solvent. The conversion ratio of cyclohexene was 84%. Cyclohexane methanol was formed in a yield of 67% and cyclohexane was formed as a hydrogenated product in a yield of 4%.

### Comparative Example 3

### [Hydroformylation of cyclohexene when a non-aqueous ionic liquid is used as the halide salt without addition of an acid]

A reaction was conducted in the same manner as in Example 8, except that an acid was not added. The conversion ratio of cyclohexene was 30%. Cyclohexane methanol was formed in a yield of 18% and cyclohexane was formed as a hydrogenated product in a yield of 1%.

The above results show that, even when a non-aqueous ionic liquid is used as the halide salt, the speed of the hydroformylation reaction is increased by the addition of an acid, and the yield of an alcohol can be improved.

### Example 9

### [Production of a polyvalent alcohol]

A reaction was conducted in the same manner as in Example 7, except that 5.0 mmol of dicyclopentadiene was used as the raw material organic compound and 10.0 mL of toluene was used as an organic solvent. The conversion ratio of dicyclopentadiene was 100%. Dicyclopentadiene dimethanol was formed in a yield of 78%, dihydrodicyclopentadiene methanol was formed in a yield of 14%, dicyclopentadiene methanol was formed in a yield of 2%, and dihydrodicyclopentadiene was formed as a hydrogenated product in a yield of 4%.

### Comparative Example 4

### [Production of a polyvalent alcohol without addition of an acid]

A reaction was conducted in the same manner as in Example 9, except that an acid was not added. The conversion ratio of dicyclopentadiene was 100%. Dicyclopentadiene dimethanol was formed in a yield of 60%, dihydrodicyclopentadiene methanol was formed in a yield of 9% and dicyclopentadiene methanol was formed in a yield of 13%. Dihydrodicyclopentadiene was formed as a hydrogenated product in a yield of 13%.

### Example 10

### [Hydroformylation of cyclohexene by using a catalyst system in which a bromide salt is used as the halide salt and phenylphosphoric acid is added]

A reaction was conducted in the same manner as in Example 2, except that 5.0 mmol of 1-butyl-3-methylimidazolium bromide was used as the halide salt and 5.0 mL of toluene was used as the solvent. The conversion ratio of cyclohexene was 95%. Cyclohexane methanol was formed in a yield of 70% and cyclohexane as a hydrogenated product was formed in a yield of 5%.

### Comparative Example 5

### [Hydroformylation of cyclohexene when a bromide salt is used as the halide salt without addition of an acid]

A reaction was conducted in the same manner as in Example 10, except that an acid was not added. The conversion ratio of cyclohexene was 51%. Cyclohexane methanol was formed in a yield of 30% and cyclohexane was formed as a hydrogenated product in a yield of 5%.

The above results show that, even when a salt other than a chloride salt is used as the halide salt, the speed of the hydroformylation reaction is increased by the addition of an acid, and the yield of a higher alcohol can be improved.

### INDUSTRIAL APPLICABILITY

According to the method of the invention, an alcohol can be obtained in a high yield within a short reaction time. This helps practical application of an environment-conscious alcohol synthesis in which, instead of carbon monoxide, carbon dioxide which is safer and inexpensive is used as a raw material.

## Claims

1. A method for producing an alcohol which comprises hydroformylation of an organic compound having an unsaturated carbon bond with carbon dioxide and hydrogen using a catalyst system comprising a ruthenium compound and a Brønsted acid containing phosphor.

2. The method of claim 1, wherein the ruthenium compound is a clustered ruthenium complex.

3. The method of claim 1 or 2, wherein the catalyst system further comprises a halide salt.

4. The method of claim 3, wherein the halide salt is a non-aqueous ionic liquid.

5. The method of any of claims 1-4, wherein the hydroformylation reaction is performed at a temperature of 100-180°C and at a pressure of 1-50 MPa.

6. The method of any of claims 1-5, wherein the organic compound is a compound having two or more unsaturated carbon bonds, and a polyvalent alcohol is produced by using the organic compound.

## Patentansprüche

1. Verfahren zur Herstellung eines Alkohols, das die Hydroformylierung einer organischen Verbindung mit einer ungesättigten Kohlenstoffbindung mit Kohlendioxid und Wasserstoff unter Verwendung eines Katalysatorsystems, das eine Rutheniumverbindung und eine phosphorhaltige Brønsted-Säure enthält, umfasst.

2. Verfahren nach Anspruch 1, bei dem die Rutheniumverbindung ein Ruthenium-Clusterkomplex ist.

3. Verfahren nach Anspruch 1 oder 2, bei dem das Katalysatorsystem ferner ein Halogenid-Salz umfasst.

4. Verfahren nach Anspruch 3, bei dem das Halogenid-Salz eine nicht-wässrige ionische Flüssigkeit ist.

5. Verfahren nach einem der Ansprüche 1-4, bei dem die Hydroformylierungsreaktion bei einer Temperatur von 100 bis 180 °C und bei einem Druck von 1 bis 50 MPa durchgeführt wird.

6. Verfahren nach einem der Ansprüche 1-5, bei dem die organische Verbindung eine Verbindung mit zwei oder mehreren ungesättigten Kohlenstoffbindungen ist, und unter Verwendung der organischen Verbindung ein mehrwertiger Alkohol hergestellt wird.

## Revendications

1. Procédé de production d'un alcool qui comprend la hydroformylation d'un composé organique ayant une liaison carbone insaturée avec du dioxyde de carbone et de l'hydrogène en utilisant un système de catalyseur comprenant un composé de ruthénium et un acide de Brønsted contenant du phosphore.

2. Procédé de la revendication 1, dans lequel le composé de ruthénium est un complexe de ruthénium sous forme agglomérée.

3. Procédé de la revendication 1 ou 2, dans lequel le système de catalyseur comprend en outre un sel d'halogénure.

4. Procédé de la revendication 3, dans lequel le sel d'halogénure est un liquide ionique non-aqueux.

5. Procédé de l'une des revendications 1 à 4, dans lequel la réaction d'hydroformylation est effectuée à une température allant de 100 à 180°C et à une pression allant de 1 à 50 MPa.

6. Procédé de l'une des revendications 1 à 5, dans lequel le composé organique est un composé ayant deux ou plusieurs liaisons carbone insaturées, et un alcool polyvalent est produit en utilisant le composé organique.
